# EUROPEAN PATENT SPECIFICATION

(11) **EP 3 689 271 B1**
(45) Date of publication and mention of the grant of the patent: **14.05.2025**
(21) Application number: 18860656.0
(22) Date of filing: 23.08.2018
(51) Int. Cl.: A61B 17/12, A61F 2/06, A61B 17/22

(54) **LEFT ATRIAL APPENDAGE OCCLUDER AND LEFT ATRIAL APPENDAGE OCCLUSION DEVICE**
VERSCHLUSS FÜR DAS LINKE HERZOHR UND VERSCHLUSSVORRICHTUNG FÜR DAS LINKE HERZOHR
OCCLUSEUR D'APPENDICE AURICULAIRE GAUCHE ET DISPOSITIF D'OCCLUSION D'APPENDICE AURICULAIRE GAUCHE

(30) Priority: 29.09.2017 CN 201710910822
(43) Date of publication of application: 05.08.2020
(73) Proprietor: Shanghai Microport Cardioadvent Co., Ltd., Shanghai 201203 (CN)
(72) Inventor: WANG, Zhen, Shanghai 201203 (CN); ZHOU, Yi, Shanghai 201203 (CN); YAO, Yao, Shanghai 201203 (CN); LI, Junfei, Shanghai 201203 (CN)
(74) Representative: Lavoix
(86) International application number: PCT/CN2018/101972
(87) International publication number: WO 2019/062403

(56) References cited:
- WO-A1-99/07289
- WO-A2-02/071977
- CN-A- 101 415 379
- CN-A- 101 460 102
- CN-A- 102 258 402
- CN-A- 105 232 187
- CN-A- 106 580 375
- US-A1- 2017 042 549
- US-A1- 2017 156 840

## Description

### TECHNICAL FIELD

The present application relates to the field of medical instruments and, in particular, to a left atrial appendage occluder and a left atrial appendage occlusion device.

### BACKGROUND

Atrial Fibrillation (AF) is the common continuous arrhythmia in clinic. The incidence of AF in the general population is 0.5-1.3%. The main hazard of AF is to promote thrombosis. Complications such as stroke and peripheral vascular embolization caused by thrombus detachment significantly increase the disability and fatality rate. Stroke is the most common and devastating complication of AF. About 15 million people worldwide suffer from stroke each year, 20-25% of which are attributed to AF. The study suggests that 60% of patients with rheumatic heart disease have a cardiogenic thrombus from the left atrial appendage, and more than 90% of thrombi in the patients with non-valvular AF are formed in the left atrial appendage. Therefore, the intervention of the left atrial appendage to prevent thromboembolism in patients with AF, especially stroke, has important theoretical and clinical significance.

Anticoagulant therapy is a standard method for preventing complications of stroke and AF at present, but has certain limitations. Therefore, the use of safer and more effective measures to prevent AF and stroke is of great significance. At present, medical interventions are mostly used to occlude the left atrial appendage. The commonly used left atrial appendage occluders comprise a single plug type occluder represented by WATCHMAN and a plug-and-disc type left atrial appendage occluder represented by AMPLATZER Cardiac Plug (ACP).

### 1. Deficiencies and disadvantages of plug type left atrial appendage occluder

The plug type left atrial appendage occluder has a nickel-titanium alloy frame with a self-expanding structure and barbs fixed around. The surface of the occluder towards atrial is covered with a porous polytetrafluoroethylene membrane for blood inflow outflow in the left atrial appendage.

When the plug type left atrial appendage occluder has been plugged into the left atrial appendage, the entrance of left atrial appendage cannot be completely occluded since the shape of the entrance of the left atrial appendage is irregular, and the occluder has a limited deformability. Therefore, it is difficult to eliminate the thrombus caused by leakage of the left atrial appendage due to AF. The left atrial appendage has different structures and depths, and in addition, a multi-cavity structure. The plug type occluder is unable to fully adapt to the anatomical structures of all left atrial appendages as well as to achieve a stable fixation.

### 2. Deficiencies and disadvantages of the plug-and-disc type left atrial appendage occluder

The plug-and-disc type left atrial appendage occluder is a two-disc type device consisting of a butterfly blade which would be placed in the left atrial appendage and a butterfly cap. The butterfly blade and the butterfly cap are connected by a concave waist. The butterfly blade is placed in the left atrial appendage to prevent the occluder from shifting. The butterfly cap seals the entrance of the left atrial appendage.

The plug-and-disc type left atrial appendage occluder has a structure in which the sealing disc and the fixed disc are integrated, and cannot be completely deformed independently. After the plug is plugged into the left atrial appendage, the disc portion adhered to the entrance of the left atrial appendage is subjected to the pull of the plug portion, so that the disc portion cannot fully fit to the entrance of the left atrial appendage, and thus it is difficult to achieve an optimal occluding effect. Moreover, since the length adjustments of the plug portion and the disc portion are limited, it is difficult to achieve the optimal fixation and blood flow blockage, and the disc structure does not adapt to different lumen shapes of the left atrial appendage.

WO02/071977A2 discloses a left atrial appendage occluder having elastic cover and anchoring substructures. The left atrial appendage occluder has H-shaped cross sections and seals the appendages by pinching an annular region of ostium tissue between the cover and the anchoring substructures.

US2017/042549A1 discloses a left atrial appendage occlusion device comprising at least one deployable tissue anchor that is configured to deploy from a constrained configuration to a deployed configuration and retractable from the deployed configuration to a retracted configuration within the body.

US2017/156840A1 discloses a medical device includes an occluder portion having an occluder eyelet and an anchor portion having an anchor eyelet, the anchor eyelet hingeably coupled to the occluder eyelet to make the anchor portion capable of moving between a retracted position and a deployed position upon the occluder portion being in an expanded position.

CN101460102A also discloses an implantable device for the left atrium of a heart, wherein at least the one portion in the secondary form has a first part facing outwardly away from the other portion and a second part which deploys first from the primary form into the secondary form and which is folded back in a direction towards the other portion on to the first part.

In short, no matter the plug type left atrial appendage occluder or the plug-and-disc type left atrial appendage occluder, once separated from the delivery system, it will be impossible for the left atrial appendage occluder to be again engaged with the delivery system for positional adjustments, if an undesired position of the left atrial appendage occluder or a leakage of occlusion is founded. This would bring a great trouble to the operator's subsequent operations.

### SUMMARY

It is an object of the present application to provide a left atrial appendage occluder and left atrial appendage occlusion device to solve the problem existed in the prior art that, once separated from the delivery system, it will be impossible for the left atrial appendage occluder to be again engaged with the delivery system for positional adjustments, if an undesired position of the left atrial appendage occluder or an leakage of occlusion is founded.

To solve the above technical problem, the application provides a left atrial appendage occluder according to claim 1.

Optionally, in the left atrial appendage occluder, the distal end of the guiding member is a blunt end.

Optionally, in the left atrial appendage occluder, the guiding member is a guide wire having a ball head or a hook at the distal end thereof.

Optionally, in the left atrial appendage occluder, the occlusion member comprises a frame, a polymer membrane covering the frame and anchors fixed on the frame, and the elastic opening is formed by means of the polymer membrane.

Optionally, in the left atrial appendage occluder, the polymer membrane comprises a plurality of membrane sheets and interstices between the plurality of membrane sheets form the elastic opening, or the polymer membrane comprises a plurality of interlaced membrane wires and interstices between the plurality of interlaced membrane wires form the elastic opening.

The present application also provides a left atrial appendage occlusion device according to claim 7.

Optionally, in the left atrial appendage occlusion device, the guiding member is a guide wire having a blunt end at a distal end thereof.

Optionally, in the left atrial appendage occlusion device, the occlusion member has an elastic opening, and the guiding member passes through the elastic opening and is removable out from the elastic opening. The elastic opening contracts upon removal of the guiding member from the elastic opening.

Optionally, in the left atrial appendage occlusion device, the occlusion member comprises a frame, a polymer membrane covering the frame and anchors fixed on the frame, and the elastic opening is formed by means of the polymer membrane.

Optionally, in the left atrial appendage occlusion device, the polymer membrane comprises a plurality of membrane sheets and interstices between the plurality of membrane sheets form the elastic opening, or the polymer membrane comprises a plurality of interlaced membrane wires and interstices between the plurality of interlaced membrane wires form the elastic opening.

In the left atrial appendage occluder and left atrial appendage occlusion device provided in the present application, after the push member is separated from the occlusion member, the guiding member is able to guide the push member to allow an easy reconnection between the occlusion member and push member for repositioning, when the push member is needed to be connected to the occlusion member again.

### BRIEF DESCRIPTION OF THE DRAWINGS

Fig. 1 schematically illustrates a status of the left atrial appendage occlusion device according to embodiment 1 of the present application.
Fig. 2 schematically illustrates another status of the left atrial appendage occlusion device according to embodiment 1 of the present application.
Fig. 3 is a structural schematic diagram of the left atrial appendage occluder according to embodiment 1 of the present application.
Fig. 4 is a structural schematic diagram of the guiding member according to embodiment 1 of the present application.
Fig. 5 is another structural schematic diagram of the guiding member according to embodiment 1 of the present application.
Fig. 6 is a structural schematic diagram of the elastic opening according to embodiment 1 of the present application.
Fig. 7 is another structural schematic diagram of the elastic opening according to embodiment 1 of the present application.
Fig. 8 is a schematic diagram showing the release of the occlusion member according to embodiment 1 of the present application.
Fig. 9 is another schematic diagram showing the release of the occlusion member according to embodiment 1 of the present application.
Fig. 10 is a structural schematic diagram of the left atrial appendage occlusion device according to embodiment 2 of the present application.
Fig. 11 is another structural schematic diagram of the left atrial appendage occlusion device according to embodiment 2 of the present application.
Fig. 12 is yet another structural schematic diagram of the left atrial appendage occlusion device according to embodiment 2 of the present application.
Fig. 13 is a structural schematic diagram of the left atrial appendage occlusion system according to embodiment 3 of the present application.

In the figures:
100, left atrial appendage occluder;
110, occlusion member;
111, frame;
112, polymer membrane;
1120, membrane sheet;
1121, membrane wire;
113, anchor;
120, guiding member;
130, push member;
140, elastic opening;
200, left atrial appendage occlusion device;
300, delivery catheter;
400, left atrial appendage occluder;
410, occlusion member;
411, frame;
412, polymer membrane;
413, anchor;
420, guiding member;
430, push member;
500, left atrial appendage occlusion device;
600, left atrial appendage occluder;
610, occlusion member;
611, frame;
6110, first frame;
6111, second frame;
6112, connector;
613, anchor;
620, guiding member;
630, push member;
700, left atrial appendage occlusion device; and
800, delivery catheter.

### DETAILED DESCRIPTION

The left atrial appendage occluder and left atrial appendage occlusion device provided in present application will be described below in further detail with reference to specific embodiments and accompanying drawings. Features and advantages of the application will be more apparent from the following detailed description and appended claims. It should be noted that the drawings are in a very simplified form and not necessarily presented to scale, for the only purpose to facilitate convenient and explicit description of embodiments of the present application. The structures shown in the accompanying drawings are often parts of the actual structures. In particular, the figures are sometimes drawn using different scales in order to give emphasis on different details.

As used herein, "proximal end" or "distal end" refers to the relative orientation, relative position, or direction of elements or actions that are relative to each other from the perspective of an operator operating the device. Yet without wishing to be limiting in any sense, the "proximal end" generally refers to the end of the medical device close to the operator during its normal operation, while the "distal end" generally refers to the end that enters into the body of the patient first.

The core idea of this application is to provide a left atrial appendage occluder, a left atrial appendage occlusion device and a left atrial appendage occlusion system. The left atrial appendage occluder includes an occlusion member and a guiding member, and the guiding member is located within the occlusion member and is removable out from the occlusion member. The proximal end of the guiding member protrudes out of the proximal end of the occlusion member. The left atrial appendage occlusion device includes the occlusion member, the guiding member and the push member. A distal end of the push member is detachably connected to the proximal end of the occlusion member. The guiding member is located within the occlusion member and push member and is removable from the occlusion member and push member. The proximal end of the guiding member protrudes out of the proximal end of the push member. The left atrial appendage occlusion system includes a delivery catheter and a left atrial appendage occlusion device. The delivery catheter is configured to establish a delivery channel, and the push member is configured to push the occlusion member along the delivery channel. The guiding member is configured, after the push member has been detached from the occlusion member, to guide the push member when the push member is needed to be connected to the occlusion member again. In present application, after the push member has been detached from the occlusion member, the guiding member is able to guide the push member to allow an easy reconnection between the occlusion member and push member for repositioning, when the push member is needed to be connected to the occlusion member again.

### EMBODIMENT 1

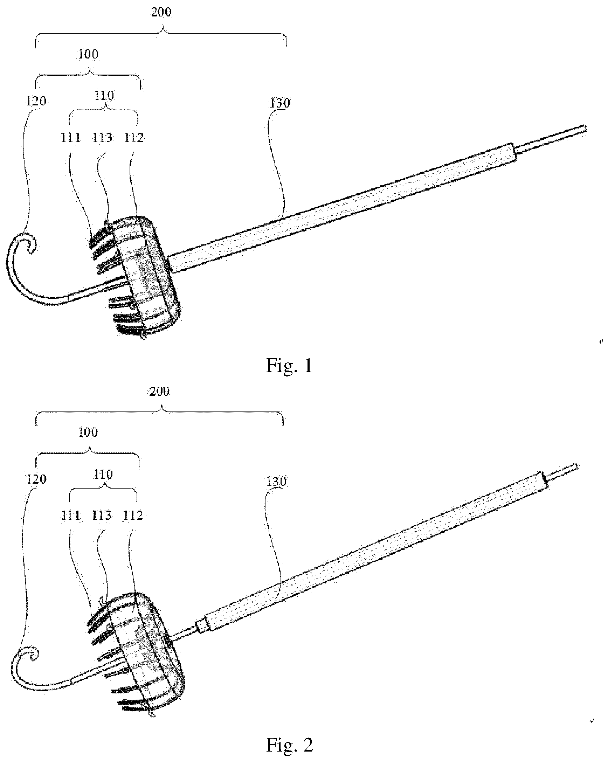

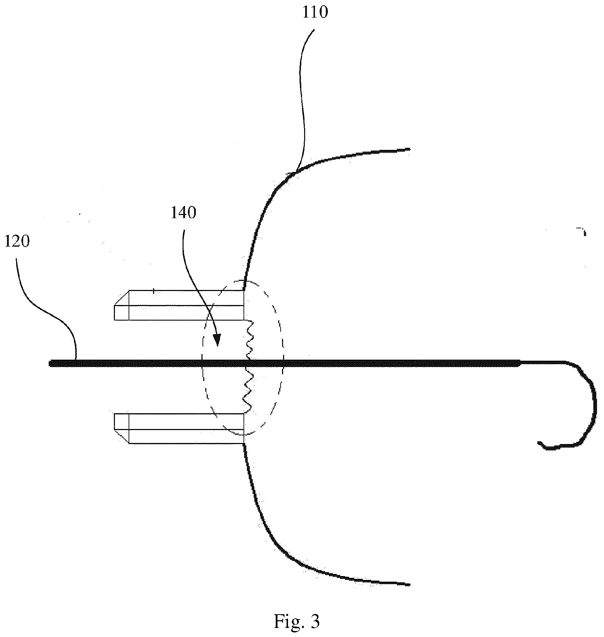

Reference is now made to Fig. 1 to Fig. 3. Fig. 1 schematically illustrates a status of the left atrial appendage occlusion device according to embodiment 1 of the present application. Fig. 2 schematically illustrates another status of the left atrial appendage occlusion device according to embodiment 1 of the present application. Fig. 3 is a structural schematic diagram of the left atrial appendage occluder according to embodiment 1 of the present application. As shown in Fig. 1 to Fig. 3, the left atrial appendage occluder 100 includes an occlusion member 110 and a guiding member 120. The guiding member 120 is located within the occlusion member 110 and is removable from the occlusion member 110 while the proximal end of the guiding member 120 protrudes out of the proximal end of the occlusion member 110. Due to the guiding member 120, the above left atrial appendage occluder 100 becomes a left atrial appendage occluder having a guiding function. After release of the left atrial appendage occluder 100 (more specifically, the occlusion member 110) by the push member, if the left atrial appendage occluder 100 is found to be released to an undesired position, or a leakage of occlusion or other situations are occurred, the push member can be easily engaged with the left atrial appendage occluder 100 (more specifically, the occlusion member 110) again under the guide of the guiding member 120, so that repositioning, withdrawal or another operations of the left atrial appendage occluder 100 are able to be achieved.

With continued reference to Figs. 1 to Fig. 3, in this embodiment, the left atrial appendage occlusion device 200 includes an occlusion member 110, a guiding member 120 and a push member 130. A distal end of the push member 130 is detachably connected to the proximal end of the occlusion member 110. The guiding member 120 is located within the occlusion member 110 and push member 130 and is removable from the occlusion member 110 and push member 130. The proximal end of the guiding member 120 protrudes out of the proximal end of the push member 130.

Specifically, the connection between the distal end of the push member 130 and the proximal end of the occlusion member 110 may be accomplished by one of the thread connection, a snap-fit connection, spline connection and plug-pin connection. For example, the distal end of the push member 130 is provided with an external thread and the proximal end of the occlusion member 110 is provided with an internal thread. Through fit of the external thread and the internal thread, the push member 130 is able to be detachably connected to the occlusion member 110. As another example, the distal end of the push member 130 is provided with an external spline and the proximal end of the occlusion member 110 is provided with an internal spline. Through fit of the external spline and the internal spline, the push member 130 is able to be detachably connected to the occlusion member 110.

In this embodiment, the guiding member 120 is a guide wire, which may be made of metal. A distal end of the guiding member 120 may be located either inside or protrude out of the occlusion member 110. Preferably, the distal end of the guiding member 120 protrudes out of the distal end of the occlusion member 110. In this case, the guiding member 120 can facilitate positioning of the occlusion member 110 during release of the occlusion member 110, so that the occlusion member 110 can be easily deployed at a suitable position.

Further, the distal end of the guiding member 120 is blunt (i.e., not sharp). In this way, when helping in positioning of the occlusion member 110 during its release, the guiding member 120 will not cause damages to the surrounding tissues, such as the left atrial appendage, thereby ensuring reliability of the left atrial appendage occluder 100 and left atrial appendage occlusion device 200.

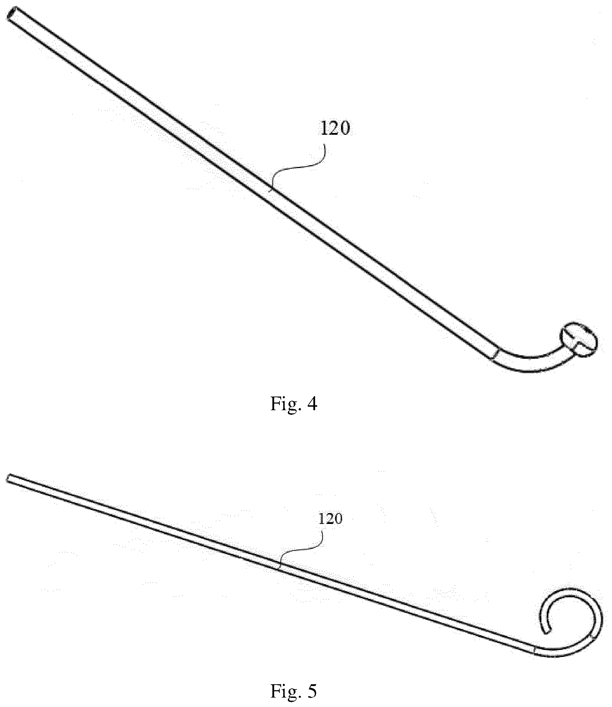

In particular, reference is now made to Fig. 4, structural schematic diagram of the guiding member according to embodiment 1 of the present application, and to Fig. 5, another structural schematic diagram of the guiding member according to embodiment 1 of the present application. As shown in Fig. 4, the distal end of the guiding member 120 may be a ball head or a body having an irregular cambered surface. In this way, the distal end of the guiding member 120 presents a cambered surface, which will not cause damages to the surrounding tissues. Besides, as shown in Fig. 5, the distal end of the guiding member 120 may also be a hook, so that no damages would be caused to the surrounding tissues. In another embodiment, the distal end of the guiding member 120 may be flexible.

With continued reference to Fig. 3, in this embodiment, the occlusion member 110 has an elastic opening 140, through which the guiding member 120 is passed. In addition, the guiding member 120 is removable from the elastic opening 140, and the elastic opening 140 contracts upon removal of the guiding member 120 from the elastic opening 140. Preferably, the elastic opening 140 will be closed after removal of the guiding member 120 from the elastic opening 140.

Specifically, the occlusion member 110 includes a frame 111, a polymer membrane 112 covering the frame 111 and anchors 113 fixed onto the frame 111. The elastic opening 140 is formed by means of the polymer membrane 112. The polymer membrane 112 may cover either an exterior or interior surface of the frame 111. The anchors 113 may be hook-shaped and made of metal or degradable polymeric material. The anchors 113 are fixed to the exterior surface of the frame 111.

The frame 111 may be umbrella-shaped and made of metal or degradable polymeric material. Specifically, the frame 111 may include a fixing part (not shown) and a plurality of frame strips connected to the fixing part. The plurality of frame strips are uniformly distributed on the fixing part. There are many interstices in frame 111 for passing the guiding member 120 therethrough. Further, the fixing part may be a hollow tube. Further, the opening of the hollow tube is covered by the polymer membrane 112 and the elastic opening is formed here by the polymer membrane 112, which can not only facilitate the passage of the guiding member 120 but also ensure a good occlusion effect. In this embodiment, the fixing part is located at the proximal end of the frame 111 (or, the occlusion member 110). Preferably, the fixing part is provided with a connecting structure, such as the thread or spline structure, to facilitate the detachable connection with the push member 130.

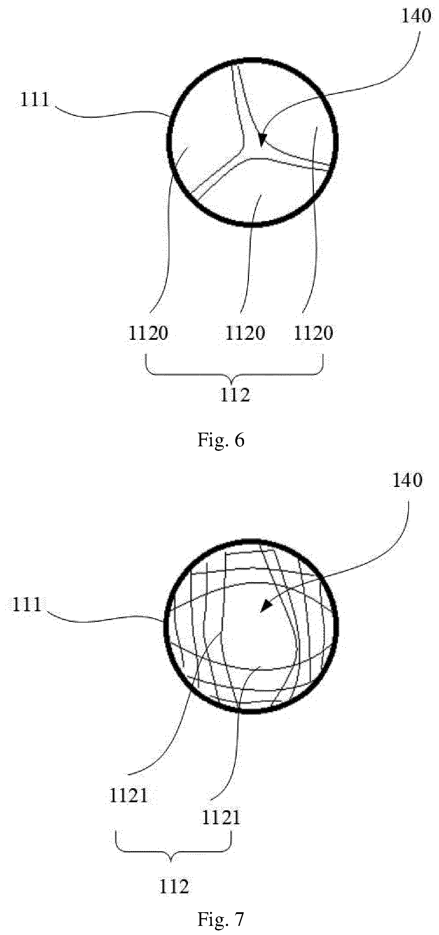

Reference is now made to Fig. 6, a structural schematic diagram of the elastic opening according to embodiment 1 of the present application, and to Fig. 7, another structural schematic diagram of the elastic opening according to embodiment 1 of the present application. As shown in Fig. 6, the elastic opening 140 may be formed as follows: the polymer membrane 112 includes a plurality of membrane sheets 1120, and interstices between the plurality of membrane sheets form the elastic opening 140. When the guiding member 120 is locating in the elastic opening shown in Fig. 6, the membrane sheets 1120 are squeezed and interstices between the plurality of membrane sheets 1120 are enlarged, thereby leading to a large elastic opening 140. Upon removal of the guiding member 120, the membrane sheets 1120 recover, and interstices between the plurality of membrane sheets 1120 contracts or closes, leading to a small elastic opening 140. Therefore, this design not only facilitates the passage of the guiding member 120 but also ensures a good occlusion effect.

Further, as shown in Fig. 7, the elastic opening 140 may also be implemented as follows : the polymer membrane 112 comprises a plurality of interlaced membrane wires 1121, and interstices between the plurality of interlaced membrane wires 1121 forms the elastic opening 140. When the guiding member 120 is locating in the elastic opening 140 shown in Fig. 7, the membrane wires 1121 are squeezed and interstices between the plurality of interlaced membrane wires 1121 are enlarged, leading to a large elastic opening 140. Upon removal of the guiding member 120, the membrane wires 1121 recover, and interstices between the plurality of interlaced membrane wires 1121 contracts or closes, leading to a small elastic opening 140. Therefore, this design not only facilitates the passage of the guiding member 120 but also ensures a good occlusion effect.

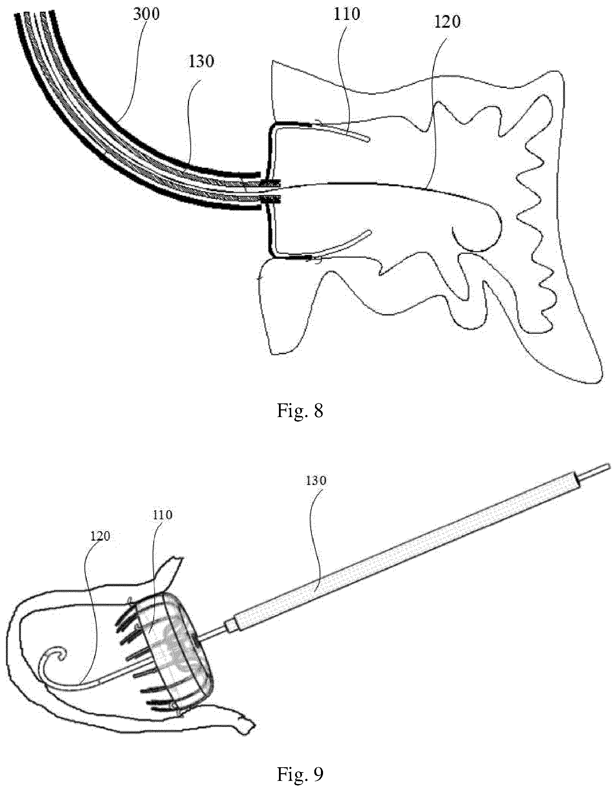

Accordingly, this embodiment also provides a left atrial appendage occlusion system. Referring to Fig. 8, the left atrial appendage occlusion system includes a delivery catheter 300 and the left atrial appendage occlusion device 200. The delivery catheter 300 is configured to establish a delivery channel, and the push member 130 is configured to push the occlusion member 110 along the delivery channel. The guiding member 120 is configured, after the push member 130 has been detached from the occlusion member 110, to guide the push member 130 when the push member 130 is needed to be connected to the occlusion member 110 again.

With continued reference to Fig. 8 and Fig. 9, occlusion of the left atrial appendage by the occlusion member 110 is achievable as follows: the delivery catheter 300 establishes a delivery channel and the guiding member 120 helps in positioning the release position of the occlusion member 110. The occlusion member 110 is pushed out of terminal of the delivery catheter 300 by the push member 130 and is released. At this time, the occlusion member 110 is plugged and tightly fitted into the left atrial appendage wall. After that, the push member 130 is detached from the occlusion member 110. Further, the push member 130 can be removable from the delivery catheter 300, meanwhile the push member 130 can also be separated from the guiding member 120. If an unsatisfactory position of the occlusion member 110 or a leakage of the occlusion is found after the separation of the push member 130 from the occlusion member 110, the push member 130 can be sleeved outside the guiding member 120 again and is reconnected to the occlusion member 110 along the guiding member 120, so that the occlusion member 110 is able to be repositioned or withdrawn. Therefore, after the push member 130 has been separated from the occlusion member 110, the guiding member 120 is able to guide the push member 130 when the push member 130 is needed to be connected to the occlusion member 110 again, so as to allow an easy reconnection of the push member 130 and the occlusion member 110 for repositioning.

### EMBODIMENT 2

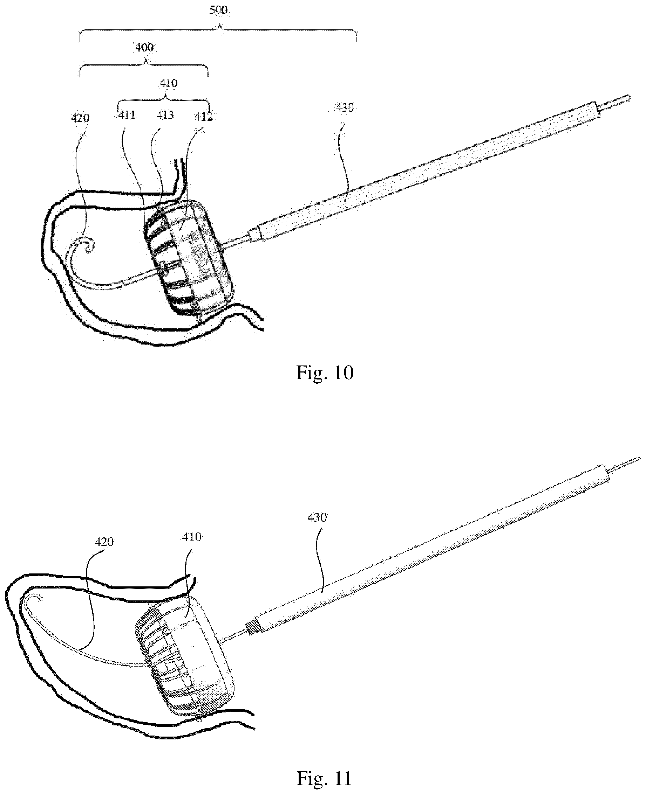

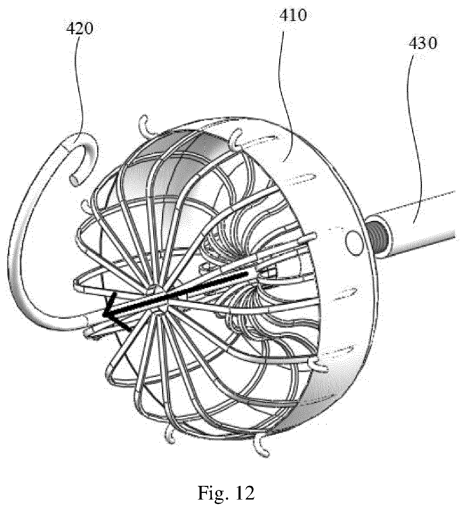

Reference is now made to Fig. 10 to Fig12. Fig. 10 is structural schematic diagram of the left atrial appendage occlusion device according to embodiment 2 of the present application. Fig. 11 is another structural schematic diagram of the left atrial appendage occlusion device according to embodiment 2 of the present application. Fig. 12 is yet another structural schematic diagram of the left atrial appendage occlusion device according to embodiment 2 of the present application. As shown in Fig. 10 to Fig. 12, in this embodiment, the left atrial appendage occluder 400 includes an occlusion member 410 and a guiding member 420.The guiding member 420 is located within the occlusion member 410 and is removable from the occlusion member 410, while the proximal end of the guiding member 420 protrudes out of the proximal end of the occlusion member 410. The left atrial appendage occlusion device 500 includes the occlusion member 410, the guiding member 420 and the push member 430. A distal end of the push member 430 is detachably connected to the proximal end of the occlusion member 410. The guiding member 420 is located within the occlusion member 410 and push member 430 and is removable from the occlusion member 410 and push member 430. The proximal end of the guiding member 420 protrudes out of the proximal end of the push member 430. The left atrial appendage occlusion system includes a delivery catheter and the left atrial appendage occlusion device 500. The delivery catheter is configured to establish a delivery channel, and the push member 430 is configured to push the occlusion member 410 along the delivery channel. The guiding member 420 is configured, after the push member 430 has been detached from the occlusion member 410, to guide the push member 430 when the push member 430 is needed to be connected to the occlusion member 410 again.

This embodiment differs from embodiment 1 in that, the frame of the occlusion member in embodiment 1 has an umbrella shape (the distal end is open) and the occlusion member thus has an umbrella shape, while the frame of the occlusion member in this embodiment has a cage shape (the distal end is closed) frame and the occlusion member thus has a cage shape.

Specifically, the occlusion member 410 includes a frame 411, a polymer membrane 412 covering the frame 411 and anchors 413 fixed onto the frame 411. An elastic opening is formed in the polymer membrane 412.

In this embodiment, the frame 411 specifically includes a first fixing part (not shown), a second fixing part (not shown) and a plurality of frame strips connected to the first fixing part and the second fixing part. Here, the first fixing part is located at the proximal end of the frame 411 and the second fixing part is located at the distal end of the frame 411. There are many interstices in frame 411 for passing the guiding member 420 therethrough. Specifically, each of the first fixing part and the second fixing part is a hollow tube. Further, the opening of the fixing part may be covered by the polymer membrane 412 and the elastic opening is formed here by the polymer membrane 412, which can not only facilitate the passage of the guiding member 420 but also ensure a good occlusion effect. In this embodiment, the first fixing part is preferably provided with a connecting structure, such as the thread or spline structure, to facilitate the detachable connection with the push member 430.

As to the guiding member 420, elastic opening, delivery catheter and other components and the relationships between components in embodiment 2, reference can be made to embodiment 1 as appropriate, description of which is omitted hereto.

### EMBODIMENT 3

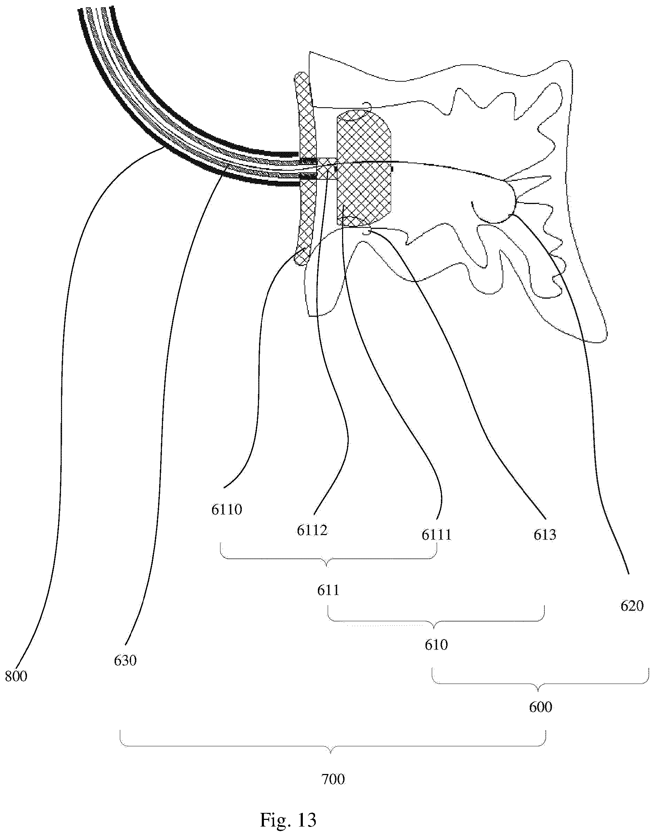

Reference is now made to Fig. 13, structural schematic diagram of the left atrial appendage occlusion system according to embodiment 3 of the present application. As shown in Fig. 13, in this embodiment, the left atrial appendage occluder 600 includes an occlusion member 610 and a guiding member 620. The guiding member 620 is located within the occlusion member 610 and is removable from the occlusion member 610, while the proximal end of the guiding member 620 protrudes out of the proximal end of the occlusion member 610. The left atrial appendage occlusion device 700 includes the occlusion member 610, the guiding member 620 and a push member 630. A distal end of the push member 630 is detachably connected to the proximal end of the occlusion member 610. The guiding member 620 is located within the occlusion member 610 and push member 630 and is removable from the occlusion member 610 and push member 630. The proximal end of the guiding member 620 protrudes out of the proximal end of the push member 630. The left atrial appendage occlusion system includes a delivery catheter 800 and the left atrial appendage occlusion device 700. The delivery catheter is configured to establish a delivery channel, and the push member 630 is configured to push the occlusion member 610 along the delivery channel. The guiding member 620 is configured to, after the push member 630 has been detached from the occlusion member 610, guide the push member 630 when the push member 630 is needed to be connected to the occlusion member 610 again.

This embodiment differs from Embodiment 1 in that, the occlusion member of Embodiment 1 comprises one frame, on which each of the polymer membrane and anchors is disposed, and is able to achieve the occlusion of the left atrial appendage as well as the fixation to the left atrial appendage, while the occlusion member in this embodiment includes two frames: a first frame and a second frame.

Specifically, the occlusion member 610 includes a frame 611, a polymer membrane covering the frame 611 and anchors 613 fixed onto the frame 611. An elastic opening is formed in the polymer membrane.

Further, the frame 611 includes a first frame 6110 and a second frame 6111. The first frame 6110 and a second frame 6111 are connected by the connector 6112. The first frame 6110 is located at the proximal end of the connector 6112, while the second frame 6111 is located at the distal end of the connector 6112. The polymer membrane covers the first frame 6110, and the anchors 613 are fixed on the second frame 6111. The first frame 6110 and the connector 6112 may be integrally formed, or may be connected by a thread structure or the like. Similarly, the second frame 6111 and the connector 6112 may be integrally formed, or may be connected by a thread structure or the like. That is, in this embodiment, occlusion of left atrial appendage can be effected by the first frame 6110 and the polymer membrane covering the first frame 6110, and the fixation of the occlusion member 610 on the left atrial appendage can be effected by the second frame 6111 and the anchors 613 fixed on the frame 6111. Preferably, the polymer membrane may also cover the second frame 6111, which makes the occlusion of left atrial appendage can also be effected by the second frame 6111 and the polymer membrane covering the second frame 6111, thereby achieving a better left atrial appendage occlusion effect.

In this embodiment, each of the first frame 6110 and the second frame 6111 has a cage shape, but in other embodiments, each of them may have an umbrella shape; or the first frame 6110 may have a cage shape while the second frame 6111 may have an umbrella shape; or the like.

In this embodiment, the first frame 6110 specifically includes a first fixing part (not shown), a second fixing part (not shown) and a plurality of frame strips connected to the first fixing part and the second fixing part. Here, the first fixing part is located at the proximal end of the first frame 6110 and the second fixing part is located at the distal end of the first frame 6110. There are many interstices in first frame 6110 for passing the guiding member 620 therethrough. Specifically, each of the first fixing part and the second fixing part is a hollow tube. Further, the opening of the first fixing part may be covered by the polymer membrane and the elastic opening is formed here by the polymer membrane, which can not only facilitate the passage of the guiding member 620 but also ensure a good occlusion effect. In this embodiment, the first fixing part is preferably provided with a connecting structure, such as the thread or spline structure, to facilitate the connection with the push member 630. Further, the second fixing part may also be provided with a connecting structure, such as the thread or spline structure, to facilitate the connection with the connector 6112.

In this embodiment, the second frame 6111 specifically includes a third fixing part (not shown), a fourth fixing part (not shown) and a plurality of frame strips connected to the third fixing part and the fourth fixing part. Here, the third fixing part is located at the proximal end of the second frame 6111 and the fourth fixing part is located at the distal end of the second frame 6111. There are many interstices in second frame 6111 for passing the guiding member 620 therethrough. Specifically, each of the third fixing part and the fourth fixing part may be a hollow tube. Further, the third fixing part may be provided with a connecting structure, such as the thread or spline structure, to facilitate the connection with the connector 6112.

In summary, in the left atrial appendage occluder, left atrial appendage occlusion device and left atrial appendage occlusion system provided in present application, after the push member is separated from the occlusion member, the guiding member is able to guide the push member to allow an easy reconnection between the occlusion member and push member for repositioning when the push member is needed to be connected to the occlusion member again.

The description presented above is merely a few preferred embodiments of the present application and does not limit the scope of the present application in any sense.

## Claims

1. A left atrial appendage occluder (100, 400, 600), comprising an occlusion member (110, 410, 610) and a guiding member (120, 420, 620), wherein the guiding member (120, 420, 620) is configured to position a push member (130, 430, 630) to the occlusion member (110, 410, 610), wherein the guiding member (120, 420, 620) is located within the occlusion member (110, 410, 610) and removable from the occlusion member (110, 410, 610), and wherein a proximal end of the guiding member (120, 420, 620) protrudes out of a proximal end of the occlusion member (110, 410, 610), wherein a distal end of the guiding member (120, 420, 620) protrudes out of a distal end of the occlusion member (110, 410, 610), **characterized in that**, the occlusion member (110, 410, 610) has an elastic opening (140), through which the guiding member (120, 420, 620) is passed, and wherein the elastic opening (140) contracts upon a removal of the guiding member (120, 420, 620) from the elastic opening (140).

2. The left atrial appendage occluder (100, 400, 600) of claim 1, wherein the distal end of the guiding member (120, 420, 620) is a blunt end.

3. The left atrial appendage occluder (100, 400, 600) of claim 2, wherein the guiding member (120, 420, 620) is a guide wire having a ball head or a hook at a distal end thereof.

4. The left atrial appendage occluder (100, 400, 600) of claim 1, wherein the occlusion member (110, 410, 610) comprises a frame (111, 411, 611), a polymer membrane (112, 412) covering the frame (111, 411, 611) and anchors (113, 413, 613) fixed on the frame (111, 411, 611), and wherein the elastic opening (140) is formed by means of the polymer membrane (112, 412).

5. The left atrial appendage occluder (100, 400, 600) of claim 4, wherein the polymer membrane (112, 412) comprises a plurality of membrane sheets (1120), and interstices between the plurality of membrane sheets (1120) form the elastic opening (140), or wherein the polymer membrane (112, 412) comprises a plurality of interlaced membrane wires (1121), and interstices between the plurality of interlaced membrane wires (1121) form the elastic opening (140).

6. A left atrial appendage occlusion device (200, 500, 700), **characterized in that**, it comprises the left atrial appendage occluder (100, 400, 600) as claimed in any one of claims 1-5, and a push member (130, 430, 630), a distal end of the push member (130, 430, 630) detachably connected to the proximal end of the occlusion member (110, 410, 610), wherein the guiding member (120, 420, 620) is configured to position the push member (130, 430, 630) to the occlusion member (110, 410, 610), wherein the guiding member (120, 420, 620) is located within the occlusion member (110, 410, 610) and push member (130, 430, 630) and movable from the occlusion member (110, 410, 610) and the push member (130, 430, 630), and wherein a proximal end of the guiding member (120, 420, 620) protrudes out of a proximal end of the push member (130, 430, 630).

7. The left atrial appendage occlusion device (200, 500, 700) of claim 6, wherein the guiding member (120, 420, 620) is a guide wire having a blunt end at a distal end thereof.

8. The left atrial appendage occlusion device (200, 500, 700) of claim 6, when not dependent on claim 4, wherein the occlusion member (110, 410, 610) comprises a frame (111, 411, 611), a polymer membrane (112, 412) covering the frame (111, 411, 611) and anchors (113, 413, 613) fixed on the frame (111, 411, 611), and wherein the elastic opening (140) is formed by means of the polymer membrane (112, 412).

9. The left atrial appendage occlusion device (200, 500, 700) of claim 8, wherein the polymer membrane (112, 412) comprises a plurality of membrane sheets (1120), and interstices between the plurality of membrane sheets (1120) form the elastic opening (140), or wherein the polymer membrane (112, 412) comprises a plurality of interlaced membrane wires (1121), and interstices between the plurality of interlaced membrane wires (1121) form the elastic opening (140).

## Patentansprüche

1. Okkluder für den linksatrialen Appendix (100, 400, 600), umfassend ein Okklusionselement (110, 410, 610) und ein Führungselement (120, 420, 620), wobei das Führungselement (120, 420, 620) so konfiguriert ist, dass es ein Druckelement (130, 430, 630) an dem Okklusionselement (110, 410, 610) positioniert, wobei das Führungselement (120, 420, 620) innerhalb des Okklusionselements (110, 410, 610) angeordnet und von dem Okklusionselement (110, 410, 610) entfernbar ist, und wobei ein proximales Ende Führungselements (120, 420, 620) aus einem proximalen Ende des Okklusionselements (110, 410, 610) herausragt, wobei ein distales Ende des Führungselements (120, 420, 620) aus einem distalen Ende des Okklusionselements (110, 410, 610) herausragt, **dadurch gekennzeichnet, dass** das Okklusionselement (110, 410, 610) eine elastische Öffnung (140) aufweist, durch die das Führungselement (120, 420, 620) hindurchgeführt wird, und wobei sich die elastische Öffnung (140) zusammenzieht, wenn das Führungselement (120, 420, 620) aus der elastischen Öffnung (140) entfernt wird.

2. Okkluder für den linksatrialen Appendix (100, 400, 600) nach Anspruch 1, wobei das distale Ende des Führungselements (120, 420, 620) ein stumpfes Ende ist.

3. Okkluder für den linksatrialen Appendix (100, 400, 600) nach Anspruch 2, wobei das Führungselement (120, 420, 620) ein Führungsdraht mit einem Kugelkopf oder einem Haken an seinem distalen Ende ist.

4. Okkluder für den linksatrialen Appendix (100, 400, 600) nach Anspruch 1, wobei das Okklusionselement (110, 410, 610) einen Rahmen (111, 411, 611), eine den Rahmen (111, 411, 611) bedeckende Polymermembran (112, 412) und an dem Rahmen (111, 411, 611) befestigte Anker (113, 413, 613) umfasst, und wobei die elastische Öffnung (140) mittels der Polymermembran (112, 412) gebildet wird.

5. Okkluder für den linksatrialen Appendix (100, 400, 600) nach Anspruch 4, wobei die Polymermembran (112, 412) eine Vielzahl von Membranblättern (1120) umfasst und Zwischenräume zwischen der Vielzahl von Membranblättern (1120) die elastische Öffnung (140) bilden oder wobei die Polymermembran (112, 412) eine Vielzahl von verflochtenen Membrandrähten (1121) umfasst und Zwischenräume zwischen der Vielzahl von verflochtenen Membrandrähten (1121) die elastische Öffnung (140) bilden.

6. Okklusionsvorrichtung für den linksatrialen Appendix (200, 500, 700), **dadurch gekennzeichnet, dass** sie den Okkluder für den linksatrialen Appendix (100, 400, 600) nach einem der Ansprüche 1-5 und ein Druckelement (130, 430, 630) umfasst, wobei ein distales Ende des Druckelements (130, 430, 630) entfernbar mit dem proximalen Ende des Okklusionselements (110, 410, 610) verbunden ist, wobei das Führungselement (120, 420, 620) so konfiguriert ist, dass es das Druckelement (130, 430, 630) an dem Okklusionselement (110, 410, 610) positioniert, wobei das Führungselement (120, 420, 620) innerhalb des Okklusionselements (110, 410, 610) und des Druckelements (130, 430, 630) angeordnet ist und von dem Okklusionselement (110, 410, 610) und dem Druckelement (130, 430, 630) weg bewegt werden kann, und wobei ein proximales Ende des Führungselements (120, 420, 620) aus einem proximalen Ende des Druckelements (130, 430, 630) herausragt.

7. Okklusionsvorrichtung für den linksatrialen Appendix (200, 500, 700) nach Anspruch 6, wobei das Führungselement (120, 420, 620) ein Führungsdraht mit einem stumpfen Ende an seinem distalen Ende ist.

8. Okklusionsvorrichtung für den linksatrialen Appendix (200, 500, 700) nach Anspruch 6, sofern sie nicht von Anspruch 4 abhängig ist, wobei das Okklusionselement (110, 410, 610) einen Rahmen (111, 411, 611), eine den Rahmen (111, 411, 611) bedeckende Polymermembran (112, 412) und an dem Rahmen (111, 411, 611) befestigte Anker (113, 413, 613) umfasst, und wobei die elastische Öffnung (140) mittels der Polymermembran (112, 412) gebildet wird.

9. Okklusionsvorrichtung für den linksatrialen Appendix (200, 500, 700) nach Anspruch 8, wobei die Polymermembran (112, 412) eine Vielzahl von Membranblättern (1120) umfasst und Zwischenräume zwischen der Vielzahl von Membranblättern (1120) die elastische Öffnung (140) bilden oder wobei die Polymermembran (112, 412) eine Vielzahl von verflochtenen Membrandrähten (1121) umfasst und Zwischenräume zwischen der Vielzahl von verflochtenen Membrandrähten (1121) die elastische Öffnung (140) bilden.

## Revendications

1. Occludeur de l'appendice auriculaire gauche (100, 400, 600), comprenant un élément d'occlusion (110, 410, 610) et un élément de guidage (120, 420, 620), dans lequel l'élément de guidage (120, 420, 620) est configuré pour positionner un élément de poussée (130, 430, 630) sur l'élément d'occlusion (110, 410, 610), dans lequel l'élément de guidage (120, 420, 620) est situé à l'intérieur de l'élément d'occlusion (110, 410, 610) et amovible de l'élément d'occlusion (110, 410, 610), et dans lequel une extrémité proximale de l'élément de guidage (120, 420, 620) dépasse de l'extrémité proximale de l'élément d'occlusion (110, 410, 610), dans lequel une extrémité distale de l'élément de guidage (120, 420, 620) dépasse de l'extrémité distale de l'élément d'occlusion (110, 410, 610), **caractérisé en ce que** l'élément d'occlusion (110, 410, 610) possède une ouverture élastique (140), à travers laquelle l'élément de guidage (120, 420, 620) est passé, et dans laquelle l'ouverture élastique (140) se contracte lors du retrait de l'élément de guidage (120, 420, 620) de l'ouverture élastique (140).

2. Occludeur de l'appendice auriculaire gauche (100, 400, 600) selon la revendication 1, dans lequel l'extrémité distale de l'élément de guidage (120, 420, 620) est une extrémité émoussée.

3. Occludeur de l'appendice auriculaire gauche (100, 400, 600) selon la revendication 2, dans lequel l'élément de guidage (120, 420, 620) est un fil de guidage doté d'une tête sphérique ou d'un crochet à son extrémité distale.

4. Occludeur de l'appendice auriculaire gauche (100, 400, 600) selon la revendication 1, dans lequel l'élément d'occlusion (110, 410, 610) comprend un cadre (111, 411, 611), une membrane polymère (112, 412) recouvrant le cadre (111, 411, 611) et des ancrages (113, 413, 613) fixés sur le cadre (111, 411, 611), et dans lequel l'ouverture élastique (140) est formée au moyen de la membrane polymère (112, 412).

5. Occludeur de l'appendice auriculaire gauche (100, 400, 600) selon la revendication 4, dans lequel la membrane polymère (112, 412) comprend une pluralité de feuilles de membrane (1120), et les interstices entre la pluralité de feuilles de membrane (1120) forment l'ouverture élastique (140), ou dans lequel la membrane polymère (112, 412) comprend une pluralité de fils de membrane entrelacés (1121), et les interstices entre la pluralité de fils de membrane entrelacés (1121) forment l'ouverture élastique (140).

6. Dispositif d'occlusion de l'appendice auriculaire gauche (200, 500, 700), **caractérisé en ce qu'**il comprend l'occludeur de l'appendice auriculaire gauche (100, 400, 600) selon l'une quelconque des revendications 1 à 5, et un élément de poussée (130, 430, 630), une extrémité distale de l'élément de poussée (130, 430, 630) étant reliée de manière amovible à l'extrémité proximale de l'élément d'occlusion (110, 410, 610), dans lequel l'élément de guidage (120, 420, 620) est configuré pour positionner l'élément de poussée (130, 430, 630) sur l'élément d'occlusion (110, 410, 610), dans lequel l'élément de guidage (120, 420, 620) est situé à l'intérieur de l'élément d'occlusion (110, 410, 610) et de l'élément de poussée (130, 430, 630) et amovible de l'élément d'occlusion (110, 410, 610) et de l'élément de poussée (130, 430, 630), et dans lequel une extrémité proximale de l'élément de guidage (120, 420, 620) dépasse d'une extrémité proximale de l'élément de poussée (130, 430, 630).

7. Dispositif d'occlusion de l'appendice auriculaire gauche (200, 500, 700) selon la revendication 6, dans lequel l'élément de guidage (120, 420, 620) est un fil de guidage dont l'extrémité distale est émoussée.

8. Dispositif d'occlusion de l'appendice auriculaire gauche (200, 500, 700) selon la revendication 6, sans dépendance à la revendication 4, dans lequel l'élément d'occlusion (110, 410, 610) comprend un cadre (111, 411, 611), une membrane polymère (112, 412) recouvrant le cadre (111, 411, 611) et des ancrages (113, 413, 613) fixés sur le cadre (111, 411, 611), et dans lequel l'ouverture élastique (140) est formée au moyen de la membrane polymère (112, 412).

9. Dispositif d'occlusion de l'appendice auriculaire gauche (200, 500, 700) selon la revendication 8, dans lequel la membrane polymère (112, 412) comprend une pluralité de feuilles de membrane (1120), et les interstices entre la pluralité de feuilles de membrane (1120) forment l'ouverture élastique (140), ou dans lequel la membrane polymère (112, 412) comprend une pluralité de fils de membrane entrelacés (1121), et les interstices entre la pluralité de fils de membrane entrelacés (1121) forment l'ouverture élastique (140).
